Europäisches Patentamt

European Patent Office

Office européen des brevets

(11) Veröffentlichungsnummer: **0 386 603 A2**

# (12) EUROPÄISCHE PATENTANMELDUNG

(21) Anmeldenummer: **90103863.8**

(22) Anmeldetag: **28.02.90**

(51) Int. Cl.⁵: **C07C 217/60, A61K 31/135, A23K 1/16**

(30) Priorität: **07.03.89 CH 841/89**

(43) Veröffentlichungstag der Anmeldung:
**12.09.90 Patentblatt 90/37**

(84) Benannte Vertragsstaaten:
**AT BE CH DE DK ES FR GB GR IT LI LU NL SE**

(71) Anmelder: **F. HOFFMANN-LA ROCHE AG**
**Postfach 3255**
**CH-4002 Basel(CH)**

(72) Erfinder: **Alig, Leo, Dr.**
**Liebrütistrasse 32**
**CH-4303 Kaiseraugst(CH)**
Erfinder: **Müller, Marcel, Dr.**
**Quellenweg 10**
**CH-4402 Frenkendorf(CH)**

(74) Vertreter: **Lederer, Franz, Dr. et al**
**Patentanwalt Dr. Franz Lederer**
**Lucile-Grahn-Strasse 22**
**D-8000 München 80(DE)**

(54) **Bisphenäthanolamine.**

(57) Das 4-[2-[Bis[(R)-β-hydroxy -3-chlorphenäthyl]amino]äthyl]phenoxyäthanol und physiologisch verträgliche Salze davon haben katabole Wirkung und können zur Behandlung der Obesitas, des Diabetes und von Zuständen, die mit erhöhtem Proteinabbau verbunden sind, oder als Futterzusatz für Masttiere verwendet werden. Sie werden ausgehend von dem oben erwähnten Ester entsprechenden Phenol hergestellt.

EP 0 386 603 A2

## Bisphenäthanolamine

Die vorliegende Erfindung betrifft neue Bisphenäthanolamine, Verfahren zu ihrer Herstellung und Pharmazeutische Präparate auf der Basis dieser Verbindungen, sowie die Verwendung dieser Verbindungen bei der Bekämpfung bzw. Verhütung von Krankheiten.

Die obigen Bisphenäthanolamine sind das 4-[2-[Bis[(R)-β-hydroxy -3-chlorphenäthyl]amino]äthyl]-phenoxyäthanol (im folgenden BAP) und physiologisch verträgliche Salze davon. Beispiele für solche Salze sind Salze von BAP mit Mineralsäuren, wie Salzsäure, Bromwasserstoffsäure, Schwefelsäure oder Phosphorsäure, oder mit organischen Säuren, wie Oxalsäure, Methansulfonsäure, Essigsäure, Propionsäure, Zitronensäure, Maleinsäure, Bernsteinsäure, Aepfelsäure, Fumarsäure, Phenylessigsäure oder Salicylsäure. Bevorzugt ist das BAP-Maleinat.

Den Phenoläther BAP und seine Salze kann man dadurch herstellen, dass man das entsprechende Phenol, 4-[2-[Bis[(R)-β-hydroxy -3-chlorphenäthyl]amino]athyl]phenol, mit einem die 2-Hydroxyäthylgruppe einführenden Mittel veräthert und gewünschtenfalls das erhaltene BAP in ein Salz überführt.

Beispiele von die 2-Hydroxyäthylgruppe einführenden Mitteln sind 2-Hydroxyäthylhalogenide, wie das Chlorid oder Bromide und 2-Hydroxyäthylsulfonate, wie das 2-Hydroxyäthylmethansulfonat. Die Reaktion eines solchen Mittels mit dem weiter oben erwähnten Ausgangsphenol kann man in an sich bekannter Weise durchführen, z.B. in einem Lösungsmittels wie Dimethylsulfoxyd (DMSO) Aceton, Tetrahydrofuran (THF) oder n-Propanol, in Gegenwart einer Base, wie Kaliumhydroxyd, Kaliumcarbonat, Kalium-t butylat oder Triäthylamin, gegebenenfalls unter Argon bei einer Temperatur bis zur Rückflusstemperatur des Reaktionsgemisches, vorzugsweise bei etwa 120°C.

Das Ausgangsphenol kann man herstellen wie beschrieben in Beispiel 1B) durch Umsetzung von Tyramin mit (R)-m-Chlorstyroloxid in einem Lösungsmittel wie DMSO, Acetonitril, THF, Dioxan oder Aethanol, bei einer Temperatur zwischen 60°C und dem Siedepunkt des Lösungsmittels.

BAP und seine Salze können als Wirkstoffe in Pharmazeutischen Präparaten zur Behandlung der Fettsucht und/oder des Diabetes mellitus, insbesondere des obesen erwachsenen Diabetikers verwendet werden. Im Tierexperiment wurde auf Verabreichung der besagten Verbindungen ein gesteigerter Katabolismus, vor allem der Fette, beobachtet. Weiterhin wurde beobachtet, dass diese Verbindungen die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen und an Streptozotocin-diabetischen Ratten haben die obigen Verbindungen einen ausgeprägten antidiabetischen Effekt, indem sie hypoglykämisch wirken und die Glukosurie vermindern. Diese Verbindungen zeigen nur eine geringe Wirkung auf Herztätigkeit und Kreislauf. Die Dosierung kann in Abhängigkeit von den individuellen Bedürfnissen des Patienten 0,5-1000 mg, vorzugsweise 2-600 mg Pro Tag für einen Erwachsenen betragen, wobei die Dosis als Einzeldosis oder in mehreren Dosen über den Tag verteilt verabreicht werden kann.

Ferner konnte mit BAP und seinen Salzen im Tierexperiment eine Erhöhung des Proteingehaltes und eine Erniedrigung des Fettgehalts im Körper nachgewiesen werden. Die besagten Verbindungen führen demnach zu einer Erhöhung der mageren Körpermasse auf Kosten des Fettanteils. Daher können sie in der Humanmedizin zur Behandlung von Zuständen, die mit erhöhtem Proteinabbau verbunden sind, z.B. bei Rekonvaleszenz nach Operationen, auch verwendet werden. Dabei sind die Verabreichungsdosen die gleichen wie bei der Behandlung der Fettsucht und/oder des Diabetes mellitus.

BAP und seine Salze können auch in der Ernährung von Masttieren, wie Rindern, Schweinen, Schafen und Geflügel, Verwendung finden. Dabei können die Verabreichungsdosen und Verabreichungsformen die gleichen sein wie für Vitamine. Die besagten Verbindungen können auch als Futterzusatz in Dosen von 0,01-100 mg/kg je nach Substanz, Tierart und Alter eingesetzt werden.

Die Pharmazeutischen Präparate enthalten den Wirkstoff zusammen mit einem verträglichen Pharmazeutischen, organischen oder anorganischen Trägermaterial, wie z.B. Wasser, Gelatine, Gummi arabicum, Lactose, Stärke, Magnesiumstearat, Talk, pflanzliche Oele, Polyalkylenglykole, Vaseline und dergleichen. Die Pharmazeutischen Präparate werden vorzugsweise oral, z.B. in Form von Tabletten, Kapseln, Pillen, Pulver, Granulaten, Lösungen, Sirupen, Suspensionen, Elixiren und dergleichen verabreicht. Die Verabreichung kann aber auch Parenteral, z.B. in Form von sterilen Lösungen, Suspensionen oder Emulsionen, erfolgen. Die Pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität von BAP wird aus den nachstehenden Versuchsresultaten deutlich:

Wirkung auf den Sauerstoffverbrauch

Gruppen von 6 männlichen Albinoratten im Gewicht von 160-180 g wurden nach 24 Stunden Fasten in Stoffwechselkäfige gesetzt. Die Käfige wurden mit konstant 6 Liter Raumluft/Minute, die bei einem Taupunkt von 11° C äquilibriert wurden belüftet. Von der Abluft wurde nach erneuter Aequilibrierung während Perioden von jeweils 14 Minuten Proben gesammelt und der Sauerstoff- und $CO_2$-Gehalt analysiert. Nach einer Anpassungszeit von 4 Stunden erhielten die Tiere entweder Placebo (5% Gummi arabicum) oder die Testsubstanz, nämlich BAP (suspendiert in 5% Gummi arabicum) per os oder intraperitoneal. Danach wurden 12 Stunden lang die Bestimmungen durchgeführt. In der nachfolgenden Tabelle ist der gemittelte Sauerstoffverbrauch nach Medikation während der ersten 3 Stunden und der gesamten Versuchsdauer (12 Stunden) als Prozentsatz des Sauerstoffverbrauchs der Anpassungsperiode (100%) angegeben, wobei entsprechende Korrekturen für Aenderungen in der Placebo-Gruppe berücksichtigt wurden.

Tabelle

| Dosis | Sauerstoffverbrauch | |
|---|---|---|
| (μM/kg) | (% vom Wert der Vorperiode) | |
| | 1. bis 3. Stunde | 1. bis 12. Stunde |
| 10 p.os | 191 | 157 |
| 3 p.os | 179 | 137 |
| 1 p.os | 156 | 122 |
| 0,3 p.os | 114 | 105 |
| 10 i.p. | 186 | 154 |
| 3 i.p. | 174 | 134 |
| 1 i.p. | 172 | 121 |
| 0,3 i.p. | 134 | 108 |

Die folgenden Beispiele erläutern die Erfindung weiter.

Beispiel 1

A) Zu einer Lösung von 520 mg 4-[2-[Bis[(R)-β-hydroxy-3-chlorphenäthyl]amino]äthyl]phenol in 7 ml n-Propanol gibt man 186 mg Kaliumhydroxyd und 650 mg 2-Bromäthanol und erwärmt die Mischung während 2 Stunden auf 120° C. Zur Aufarbeitung wird auf Eiswasser gegossen und mit Aethylacetat extrahiert. Der Extrakt ergibt nach Waschen mit Wasser, Trocknen über $Na_2SO_4$ und Abdampfen des Lösungsmittels 600 mg rohes Oel. Nach Chromatographie an 40 g Silicagel mit Chloroform/n-Propanol/wässrigem Ammoniak (1000:10:1) erhält man 290 mg BAP, $[\alpha]_D^{20}$ = -66° (c = 1,0 in Methanol); $\epsilon_{225}$ = 14080.

B) Zur Herstellung des Ausgangsphenols werden 10,0 g Tyramin und 8,8 g (R)-m-Chlorstyroloxid in 300 ml DMSO 24 Stunden auf 100° C erhitzt. Dann werden nochmals 17,5 g (R)-m-Chlorstyroloxid zugegeben, die man weitere 24 Stunden auf 100° C erwärmt. Das Lösungsmittel wird dann im Hochvakuum abgedampft und der Rückstand wird mit Chloroform/n-Pro panol/gesättigtem Ammoniak (1000:5:0,2) auf 1 kg Silicagel chromtographiert. Die einheitlichen Fraktionen werden vereinigt. Man erhält 13 g des erwünschten Phenols, $[a]_D^{20}$ = -22° (c = 0,8 in Methanol).

Beispiel 2

Es werden in üblicher Weise Tabletten folgender Zusammensetzung hergestellt:

| BAP | 250 mg |
|---|---|
| Lactose | 200 mg |
| Maisstärke | 300 mg |
| Maisstärkepaste | 50 mg |
| Calciumstearat | 5 mg |
| Dicalciumphosphat | 45 mg |

**Ansprüche**

1. 4-[2-[Bis[(R)-β-hydroxy-3-chlorphenäthyl]amino]-äthyl]phenoxyäthanol und physiologisch verträgliche Salze davon.

2. Verbindungen nach Anspruch 1 als therapeutische Wirkstoffen insbesondere zur Behandlung der Fettsucht, des Diabetes mellitus und von Zuständen, die mit erhöhtem Proteinabbau verbunden sind.

3. Pharmazeutische Präparate, gekennzeichnet durch einen Gehalt an einer Verbindung nach Anpruch 1.

4. Futtermittel für Masttiere enthaltend eine Verbindung nach Anspruch 1.

5. Verfahren zur Herstellung des 4-[2-[Bis[(R)-β-hydroxy -3-chlorphenäthyl]amino]äthyl]phenoxyäthanols und von physiologisch verträglichen Salzen davon, dadurch gekennzeichnet, dass man das 4-[2-[Bis[(R)-β-hydroxy-3-chlorphenäthyl]amino]äthyl]phenol mit einem die 2-Hydroxyäthylgruppe einführenden Mittel veräthert und gewünschtenfalls die erhaltene Verbindung in ein Salz überführt.

6. Verwendung einer Verbindung nach Anspruch 1 zur Herstellung von pharmazeutischen Präparaten zur Behandlung der Fettsucht, des Diabetes mellitus und von Zuständen, die mit erhöhtem Proteinabbau verbunden sind.

Patentansprüche für folgende Vertragsstaaten: GR, ES.

1. Verfahren zur Herstellung des 4-[2-[Bis[(R)-β-hydroxy -3-chlorphenäthyl]amino]äthyl]phenoxyäthanols und von physiologisch verträglichen Salzen davon, dadurch gekennzeichnet, dass man das 4-[2-[Bis[(R)-β-hydroxy-3-chlorphenäthyl]amino]äthyl]phenol mit einem die 2-Hydroxyäthylgruppe einführenden Mittel veräthert und gewünschtenfalls die erhaltene Verbindung in ein Salz überführt.

2. Verfahren nach Anspruch 1, dadurch gekennzeichnet, dass man das 4-[2-[Bis[(R)-β-hydroxy-3-chlorphenäthyl]amino]äthyl]phenol mit einem 2-Hydroxyäthylhalogenid oder 2-Hydroxyäthylsulfonat in einem Lösungsmittel in Gegenwart einer Base bei einer Temperatur bis zu Rückflusstemperatur des Reaktionsgemisches veräthert.

3. Verfahren zur Herstellung von Arzneimitteln, insbesondere zur Behandlung der Fettsucht, des Diabetes mellitus und von Zuständen, die mit erhöhtem Proteinabbau verbunden sind, dadurch gekennzeichnet, dass man eine Verbindung nach Anspruch 1 in eine galenische Darreichungsform bringt.

4. Verwendung einer Verbindung nach Anspruch 1 zur Herstellung von Pharmazeutischen Präparaten zur Behandlung der Fettsucht, des Diabetes mellitus und von Zuständen, die mit erhöhtem Proteinabbau verbunden sind.